# FASCICULE DE BREVET EUROPEEN

(11) **EP 3 188 780 B1**
(45) Date de publication et mention de la délivrance du brevet: **03.07.2019**
(21) Numéro de dépôt: 15788103.8
(22) Date de dépôt: 01.09.2015
(51) Int. Cl.: A61M 11/00, A61M 15/08, A61M 11/08, A61M 15/00

(54) **DISPOSITIF DE PULVÉRISATION NASALE DE PRODUIT FLUIDE**
NASENSPRAYVORRICHTUNG ZUR VERABREICHUNG VON FLÜSSIGPRODUKTEN
DEVICE FOR NASAL SPRAYING OF FLUID PRODUCT

(30) Priorité: 02.09.2014 FR 1458181
(43) Date de publication de la demande: 12.07.2017
(73) Titulaire: Université de Tours, 37020 Tours Cedex 1 (FR)
(72) Inventeur: FRANCIS, Mirvatte, F-37000 Tours (FR); VECELLIO-NONE, Laurent, F-37170 Chambray Les Tours (FR)
(74) Mandataire: CAPRI
(86) Numéro de dépôt international: PCT/FR2015/052306
(87) Numéro de publication internationale: WO 2016/034803

(56) Documents cités:
- EP-A2- 1 023 911
- WO-A2-2012/024595
- DE-A1- 1 625 238
- US-A- 2 772 117
- US-A1- 2010 030 188

## Description

La présente invention concerne un dispositif de pulvérisation nasale d'un produit fluide, en particulier un produit fluide pharmaceutique.

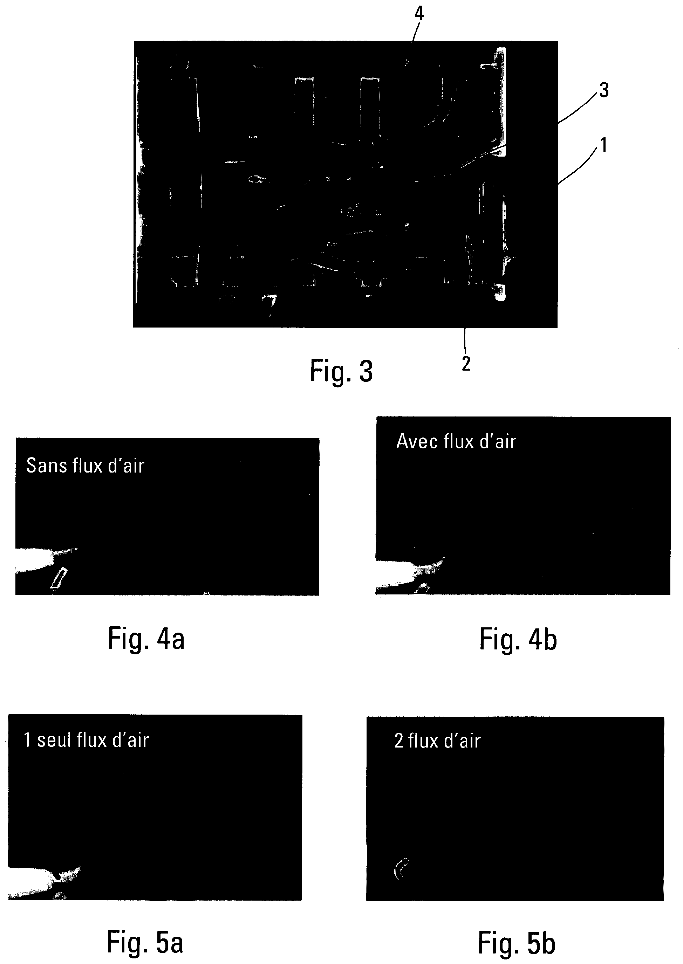

Les cavités nasales sont dissociées de part et d'autre de manière longitudinale par le septum. Chacune des cavités nasales se compose d'abord du nez puis ensuite d'une valve nasale. Cette valve nasale possède une géométrie particulière. Elle s'étend sur environ 1 cm de profondeur et a une section longitudinale verticale d'environ 3 à 4 cm et une largeur d'environ 1 à 3 mm. Après le passage de la valve nasale, les cavités nasales sont composées d'une plus grande cavité (environ 7 cm de hauteur sur 2 à 3 cm de largeur). Les cornets font face à la valve nasale. Au-dessus des cornets est situé le plafond de la cavité nasale, comprenant les ethmoïdes, le bulbe olfactif et le nerf olfactif. La figure 3, qui illustre une imagerie d'un modèle anatomique de cavité nasale, montre le nez 1, la valve nasale 2, les cornets 3 et les ethmoïdes 4.

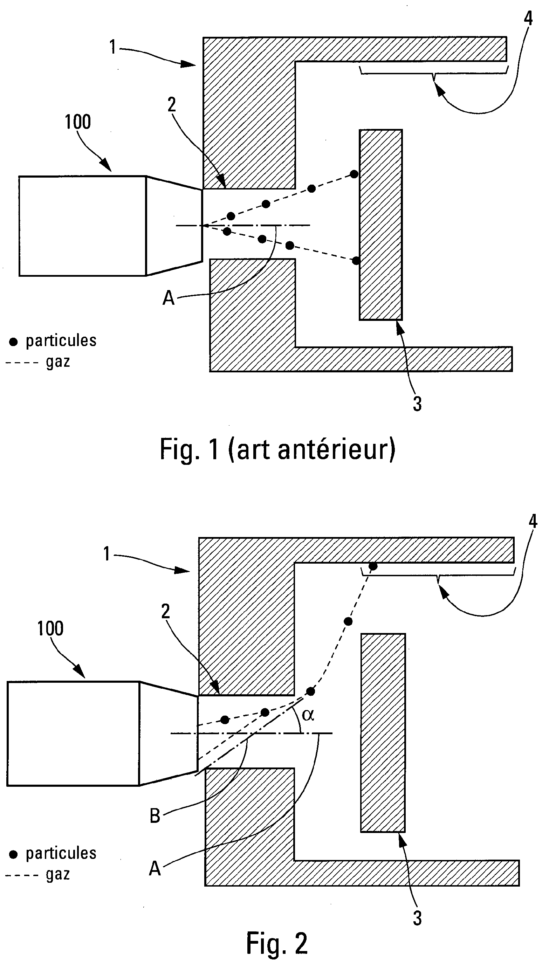

Les dispositifs de pulvérisation nasale de produit fluide pharmaceutique sont bien connus dans l'état de la technique. Ces dispositifs génèrent un spray rectiligne avec un angle solide, qui, comme visible sur la figure 1 (qui représente schématiquement une pulvérisation nasale selon l'art antérieur), va pénétrer axialement dans la narine de l'utilisateur. La figure 1 montre un inconvénient majeur des dispositifs de l'état de la technique. En effet, le dispositif de pulvérisation nasale 100 n'étant pas ou peu invasif, celui-ci ne franchit pas la valve nasale 2. Ainsi, de par l'anatomie de la valve nasale 2 et de l'emplacement protectif des cornets 3, la trajectoire axiale ou rectiligne des particules du spray ne permet pas d'atteindre le plafond de la cavité nasale, et notamment les ethmoïdes 4.

Les documents WO03026559, WO02068031 et WO2012024595 divulguent des dispositifs de l'état de la technique. Le dispositif du document WO03026559 utilise un mouvement vortex de la trajectoire des particules pour améliorer le ciblage distal des cavités nasales. Ce système requiert des particules fines de l'ordre de 2 à 50 micromètres. Le système du document WO02068031 utilise également des particules fines (aérosol) pour améliorer le ciblage des éthmoïdes en limitant le dépôt pulmonaire par la synchronisation de la génération de l'aérosol durant la phase expiratoire du patient. Le document WO2012024595 décrit un flux d'air périphérique rotationnel en spirale qui entoure axialement le spray afin de l'entrainer, sans toutefois le dévier de sa direction axiale. Le cône du spray reste donc symétrique par rapport à sa direction axiale.

Ces dispositifs antérieurs sont complexes à mettre en oeuvre, et impliquent notamment des modifications substantielles des dispositifs de pulvérisation nasale standard. Par ailleurs, l'utilisation de fines particules pose le problème de l'efficacité totale du dépôt dans les cavités nasales. En effet une partie des fines particules n'est généralement pas déposée dans la totalité des cavités nasales (voir notamment la publication intitulée "Nasally inhaled pulsating aerosols: lung, sinus and nose déposition" de Moller W, Saba GK, Haussinger K, Becker S, Keller M, Schuschnig U, Rhinology, 2011 Aug;49(3):286-91), rendant ainsi moins efficace le dispositif et donc le traitement médical.

La présente invention a pour but de fournir un dispositif de pulvérisation nasale de produit fluide qui ne reproduit pas les inconvénients susmentionnés.

La présente invention a aussi pour but de fournir un dispositif de pulvérisation nasale de produit fluide qui améliore la distribution du produit fluide pulvérisé dans la narine de l'utilisateur.

La présente invention a également pour but de fournir un dispositif de pulvérisation nasale de produit fluide qui est simple et peu coûteux à fabriquer et à assembler.

La présente invention a donc pour objet un dispositif de pulvérisation nasale de produit fluide comportant un réservoir contenant au moins une dose de produit fluide, un système de distribution pour pulvériser, à chaque actionnement du dispositif, une dose de produit à travers un orifice de pulvérisation, générant un spray s'étendant axialement à partir dudit orifice de pulvérisation, ledit dispositif comprenant un système de génération de flux secondaire actionné simultanément audit système de distribution, ledit système de génération de flux secondaire comportant un canal de flux secondaire pourvu d'un orifice de sortie, ledit orifice de sortie étant disposé en aval dudit orifice de pulvérisation dans la direction axiale dudit spray, et ledit flux secondaire s'étendant à partir dudit orifice de sortie selon un axe B formant un angle par rapport à l'axe A dudit spray et coupant ledit axe A, pour ainsi déformer et/ou dévier ledit spray.

Avantageusement, ledit flux secondaire dudit système de génération de flux secondaire est formé par une source de gaz externe, notamment de gaz comprimé.

En variante, ledit flux secondaire dudit système de génération de flux secondaire est formé par le flux d'inhalation de l'utilisateur.

Avantageusement, ledit flux secondaire dudit système de génération de flux secondaire est constitué de gaz, notamment d'air.

En variante, ledit flux secondaire dudit système de génération de flux secondaire comporte du gaz, notamment de l'air, mélangé avec du produit fluide, notamment des particules de poudre.

Avantageusement, ledit angle α est compris entre 10° et 90°, avantageusement entre 20° et 80°, de préférence entre 30° et 70°, notamment environ 35°.

Avantageusement, le diamètre dudit orifice de pulvérisation est sensiblement identique au diamètre dudit orifice de sortie, avantageusement égal à environ 2 mm.

En variante, le diamètre dudit orifice de pulvérisation est inférieur au diamètre dudit orifice de sortie.

Avantageusement, le diamètre dudit orifice de pulvérisation est de 0,3 mm et le diamètre dudit orifice de sortie est de 0,4 mm.

Avantageusement, ledit canal de flux secondaire dudit système de génération de flux secondaire comporte un orifice d'entrée.

Avantageusement, ledit orifice d'entrée est relié à une source de gaz, notamment d'air, comprimé.

En variante, ledit orifice d'entrée est relié à l'atmosphère.

Avantageusement, ledit produit fluide est un médicament sous forme de poudre.

En variante, ledit produit fluide est un médicament sous forme liquide.

Avantageusement, ledit spray est composé de particules dont le diamètre est compris entre 1µm et 500µm.

Avantageusement, ledit flux secondaire déforme et/ou dévie ledit spray de manière latéral dans la narine de l'utilisateur.

Avantageusement, lors de l'actionnement, ledit orifice de pulvérisation est inséré dans la narine d'une profondeur comprise entre 15 et 30 mm.

Avantageusement, pour un modèle d'homme, au moins 10%, avantageusement environ 50%, du spray est déposé sur les ethmoïdes.

Avantageusement, pour un modèle de femme, au moins 2%, avantageusement environ 20%, du spray est déposé sur les ethmoïdes.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue très schématique d'une pulvérisation nasale réalisée avec un dispositif de pulvérisation nasale selon l'art antérieur,
- la figure 2 est une vue similaire à celle de la figure 1, montrant une pulvérisation nasale réalisée avec un dispositif de pulvérisation nasale selon la présente invention,
- la figure 3 est une vue schématique montrant une imagerie d'un modèle anatomique de cavité nasale,
- les figures 4a et 4b montrent les formes du spray, respectivement avec un dispositif de pulvérisation nasale selon l'art antérieur et avec un dispositif de pulvérisation nasale selon la présente invention,
- les figures 5a et 5b montrent les formes du spray avec un dispositif de pulvérisation nasale selon deux modes de réalisations de la présente invention,
- les figures 6 et 7 sont des vues très schématiques d'un dispositif de pulvérisation nasale selon un mode de réalisation avantageux de la présente invention, respectivement avant et pendant la pulvérisation du produit fluide,
- les figures 8 et 9 sont des vues similaires à celles des figures 6 et 7, montrant un autre mode de réalisation avantageux de la présente invention,
- les figures 10 et 11 sont des vues similaires à celles des figures 6 et 7, montrant encore un autre mode de réalisation avantageux de la présente invention,
- les figures 12 et 13 sont des vues similaires à celles des figures 6 et 7, montrant encore un autre mode de réalisation avantageux de la présente invention,
- La figure 14 est un graphique représentant les dépôts de spray dans les différentes zones de la narine pour un homme et pour une femme, avec un dispositif réalisé et orienté comme sur les figures 12 et 13, c'est-à-dire avec le flux d'air déviant le spray vers le haut,
- La figure 15 est une représentation schématique d'un dispositif réalisé selon les figures 12 et 13, mais orienté différemment dans la narine, avec le flux d'air déviant le spray de manière latérale,
- La figure 16 est un graphique représentant les dépôts de spray dans les différentes zones de la narine pour un modèle d'homme, avec un dispositif réalisé et orienté comme sur la figure 15, et comparant deux profondeurs d'insertion dans la narine, et
- La figure 17 est le même graphique que celui de la figure 16, pour un modèle de femme.

Dans la description, le terme "axial" se réfère à la direction longitudinale et rectiligne du spray tel qu'il est expulsé à travers l'orifice de pulvérisation du dispositif, c'est-à-dire l'axe A sur les figures.

L'invention s'applique plus particulièrement à des dispositifs de pulvérisation nasale du type unidose (dispositif ne distribuant qu'une seule dose) ou bidose (dispositif ne distribuant que deux doses), mais elle pourrait aussi s'appliquer à un multidose (dispositif distribuant plus de deux doses). Un tel dispositif de pulvérisation nasale comporte généralement un réservoir contenant une, deux ou plusieurs doses de produit fluide à distribuer par pulvérisation nasale.

Dans le cas d'un unidose, une dose unique est distribuée en un seul actionnement du dispositif. Dans le cas des bidoses, contenant deux doses, et des multidoses, contenant plus de deux de doses, ces doses sont distribuées successivement lors d'actionnements successifs du dispositif.

Un système de distribution, qui peut être une pompe, une valve aérosol, un piston coulissant dans le réservoir ou une chasse d'air, est généralement utilisé pour transférer une dose à chaque actionnement vers un orifice de pulvérisation, de préférence disposé à l'extrémité axiale d'une tête de pulvérisation nasale.

Le système de distribution peut générer le spray, par exemple en aérosolisant une poudre ou un liquide dans un flux d'air ou de gaz.

En variante la tête de pulvérisation peut comporter un profil de pulvérisation prévu juste en amont dudit orifice de pulvérisation, qui va générer un spray à travers ledit orifice de pulvérisation. Ce profil de pulvérisation peut être de tout type approprié, par exemple avec des canaux non radiaux menant vers une chambre de tourbillonnement directement reliée audit orifice de pulvérisation, provoquant ainsi un tourbillonnement du produit fluide juste avant son expulsion sous forme de spray à travers ledit orifice de pulvérisation. Il est à noter qu'un tel profil de pulvérisation en amont de l'orifice de pulvérisation n'est pas obligatoire, et que le spray peut être généré d'une quelconque manière appropriée, au niveau dudit orifice de pulvérisation, ou en amont de celui-ci.

Le spray ainsi généré est expulsé à travers ledit orifice de pulvérisation selon une direction axiale, et présente généralement une forme de cône symétrique s'évasant à partir dudit orifice de pulvérisation.

Selon l'invention, le dispositif de pulvérisation nasale est apte à déposer une quantité de produit fluide dans différentes zones des cavités nasales, et plus particulièrement sur le plafond des cavités nasales, comprenant notamment les ethmoïdes, le bulbe olfactif et le nerf olfactif. Pour ce faire, le dispositif selon l'invention comporte d'une part un système de distribution 20 pour pulvériser, à chaque actionnement du dispositif, une dose D de produit à travers un orifice de pulvérisation 35, générant un spray s'étendant axialement à partir dudit orifice de pulvérisation 35. D'autre part, le dispositif comporte un système de génération de flux secondaire 40 actionné simultanément audit système de distribution 20. Ce système de génération de flux secondaire 40 comporte un canal de flux secondaire 41 pourvu d'un orifice de sortie 45. Selon l'invention, ledit orifice de sortie 45 est disposé en aval dudit orifice de pulvérisation 35 dans la direction axiale dudit spray, et ledit flux secondaire s'étend à partir dudit orifice de sortie 45 selon un axe B formant un angle α par rapport à l'axe A dudit spray et coupant ledit axe A dudit spray, pour ainsi déformer et/ou dévier ledit spray. De préférence, le dispositif de pulvérisation nasale est orienté de manière appropriée par rapport à la narine pour permettre aux particules de spray d'atteindre les zones ciblées. Eventuellement, des moyens d'orientation peuvent être prévus pour inciter l'utilisateur à insérer le dispositif dans la narine avec une bonne orientation.

Le dispositif de l'invention comprend donc un premier générateur produisant des particules avec une vitesse initiale sensiblement rectiligne et un second générateur, qui peut être actif ou passif, de flux secondaire, permettant la déviation de la trajectoire rectiligne de l'aérosol après sa génération. Le second générateur est placé en aval du premier générateur (dans le sens de distribution), de sorte qu'il n'agit sur le spray qu'après que celui-ci est expulsé de l'orifice de pulvérisation 35. L'idée consiste à obtenir une trajectoire non rectiligne des particules du spray afin de contourner les cornets et atteindre les éthmoïdes, comme représenté schématiquement sur la figure 2. Le flux secondaire entre en interaction avec la trajectoire des particules du spray afin de les faire dévier, notamment selon une trajectoire vers le haut dans l'orientation de la figure 2. Ainsi, ledit spray, qui est sensiblement symétrique par rapport à l'axe A lorsqu'il sort de l'orifice de pulvérisation 35, est déformé et/ou dévié par ledit flux secondaire, de sorte qu'il devient asymétrique, ce qui distingue la présente invention de l'art antérieur, et notamment du document WO2012024595.

L'angle α formé entre la trajectoire de ce flux secondaire (l'axe B) et la trajectoire des particules du spray produit par le premier générateur (l'axe A) est avantageusement compris entre 10° et 90°, notamment entre 20° et 80°, de préférence entre 30° et 70°. Il est à noter qu'une trop forte déviation du spray dans la valve nasale 2 pourrait avoir pour effet un dépôt de produit fluide majoritairement, voire totalement, dans la valve nasale, ce qui ne serait pas souhaitable. Les paramètres du flux secondaire, et en particulier le débit, la vitesse et l'angle α de ce flux secondaire, doivent donc être ajustés et adaptés au spray généré par le système de distribution 20. Ainsi, selon la nature de ce système de distribution 20, les paramètres du spray expulsé à travers l'orifice de pulvérisation 35, tel que la vitesse des particules et leur densité, peuvent varier, et par conséquent les paramètres du flux secondaire devront être adaptés à chaque situation particulière.

Pour vérifier le fonctionnement de l'invention, la forme d'un spray, c'est-à-dire une plume, à base de poudre aérosolisée a été filmée avec et sans ajout d'un flux secondaire. L'orientation du flux secondaire par rapport à l'axe du spray était d'environ 80°. Le débit du flux secondaire était réglé à 15 L/min et le diamètre de l'orifice de sortie du canal de flux secondaire était de 0,9 mm, donnant une vitesse de sortie du flux d'air secondaire de 393 m/s.

La figure 4a montre une photo de la plume classique, sans ajout du flux secondaire. La figure 4b montre la forme de la plume en ajoutant le flux secondaire selon l'invention. Ces photos montrent bien un changement significative de la forme de la plume, et les particules du spray atteignent des endroits plus éloignés de la cavité nasale qu'avec une plume classique, comme représenté très schématiquement sur les figures 1 et 2.

Pour vérifier si la déviation de la trajectoire des particules du spray permet de cibler les éthmoïdes, un dispositif de pulvérisation nasale 100 du type unidose poudre a été chargé avec de la fluorescéine et testé sur deux modèles de cavités nasales, un modèle de type homme et un modèle de type femme. La vitesse de sortie moyenne des sprays était de 33 m/s, avec un minimum de 22 m/s et un maximum de 52 m/s (avec le dispositif de pulvérisation nasale utilisé pour ce test, cette vitesse était dépendante de la force d'actionnement du dispositif). Pour le flux secondaire, on a ajouté à proximité de l'orifice de pulvérisation un tuyau incliné, ce qui a permis de générer le flux secondaire avec une de vitesse de 393 m/s et une orientation d'environ 80°.

Une comparaison du dépôt de particules de poudre dans les zones cibles a été réalisée dans les deux cas. Pour les deux tests, les mêmes configurations ont été choisies, c'est-à-dire que le dispositif de pulvérisation était orienté dans les deux cas à environ 30° dans la narine et la pénétration dans la narine était similaire. On a constaté qu'il y a bien un dépôt significatif dans les hauts des éthmoïdes en ajoutant un flux secondaire. Par contre, sans ajout du flux secondaire, les particules du spray n'atteignent pas les ethmoïdes et le dépôt se trouve principalement dans le bas des cornets et sur les planchers. Le dosage de la masse déposée dans les hauts des cornets par rapport au total déposé était de 3% en ajoutant un flux secondaire, alors qu'il était de 0% avec le dispositif classique, sans ajout du flux secondaire.

D'autres tests ont étés effectués sur le modèle homme en comparant le dépôt de poudre obtenu avec le dispositif classique et ceux obtenus en ajoutant des flux secondaires ayant des vitesses différentes (65.8 m/s et 262 m/s). Le dépôt dans les éthmoïdes sans flux secondaire était de 0%, le dépôt avec un flux secondaire ayant une vitesse de 65.8 m/s était de 3.6%, et le dépôt avec un flux secondaire ayant une vitesse de 262 m/s était de 10%. En d'autre terme, plus la vitesse de l'air est importante par rapport à celle des particules du spray, plus ces particules suivront la trajectoire du flux secondaire. Il est donc possible d'augmenter le dépôt dans les éthmoïdes en augmentant la vitesse du flux secondaire par rapport à celle du spray. Pour éviter d'avoir à utiliser des flux secondaires trop puissants, qui pourraient s'avérer gênants pour les utilisateurs, il serait souhaitable de réduire la vitesse du spray, ce qui permettrait d'obtenir les mêmes effets avec un flux secondaire moins puissant.

Par ailleurs, en ajoutant plusieurs flux secondaires parallèles, la probabilité que les particules de spray suivent la trajectoire souhaitée augmente. Les figures 5a et 5b montrent une comparaison entre la forme de la plume, toujours obtenu avec un dispositif de pulvérisation du type unidose poudre, en ajoutant d'une part un seul flux secondaire (figure 5a), et en ajoutant d'autre part deux flux secondaires parallèles (figure 5b). Une comparaison de la partie basse des plumes montre qu'en ajoutant plusieurs flux secondaires, une plus grande partie des particules de spray suit la trajectoire souhaitée. En effet, sur la figure 5b, il y a moins de particules de spray présentes dans la zone inférieure. Cette situation correspond également à l'utilisation d'un flux d'air de type « lame » de flux d'air. La géométrie de l'orifice du flux d'air peut avoir une section circulaire mais également elliptique ou encore rectangulaire.

Un autre test a été réalisé avec un nébuliseur. En ajoutant un flux secondaire (orienté à environ 80°) à un nébuliseur produisant des fines particules (1µm à 10µm), le flux secondaire ayant une vitesse de sortie supérieure à celle du spray (20 m/s contre 16 m/s) et ayant une section de l'orifice de sortie du même ordre que celle de l'orifice de pulvérisation, la trajectoire et la forme de la plume a été fortement impactée par le flux d'air. En effet le dépôt dans les cornets a augmenté significativement de 2.4% à 48.48%, et le dépôt dans les planchers a diminué de 72.29% à 4.9 %.

En ajoutant un flux secondaire ayant une vitesse de 26 m/s mais sortant d'un tuyau ayant une section de l'orifice de sortie (2,2 mm²) inférieure à celle de l'orifice de pulvérisation (3,2 mm²), seule une partie des particules de spray a été déviée. En effet, le dépôt dans les cornets n'a dans ce cas augmenté que de 4.98 %.

Il semble donc souhaitable d'ajouter un flux secondaire ayant d'une part une vitesse de sortie supérieure à celle des particules de spray. D'autre part, la section de l'orifice de sortie du système de génération de flux secondaire devrait être au minimum du même ordre de celle de l'orifice de pulvérisation du spray, pour qu'une majeure partie des aérosols soit déviée.

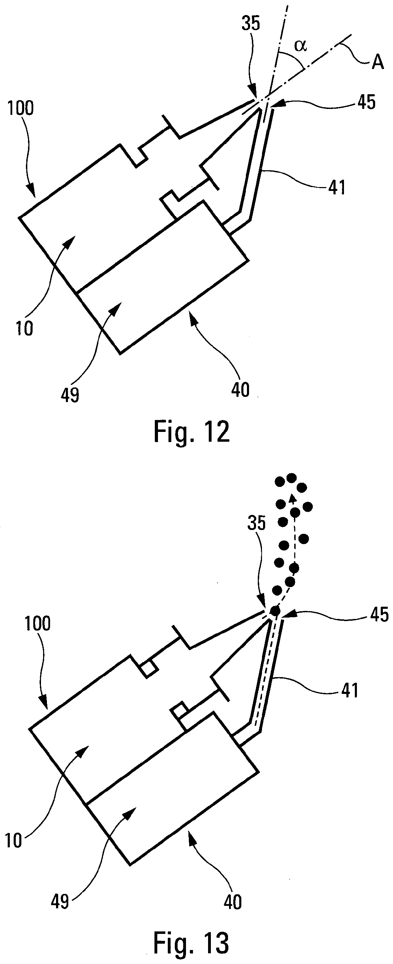

Un mode de réalisation de cette invention est représenté sur les figures 12 et 13. Dans cette configuration, le dispositif de pulvérisation nasale 100 est un pulvérisateur nasal de liquide muni d'un orifice de pulvérisation 35. Le médicament liquide est contenu dans le réservoir 10 et des doses de médicaments sous forme de spray de particules sont produites au niveau de l'orifice de pulvérisation 35, typiquement au moyen d'une valve doseuse fonctionnant avec un gaz propulseur (non représentée sur ces figures). Le dispositif de pulvérisation nasale 100 est solidaire d'un système de génération de flux secondaire 40 contenant un gaz propulseur, typiquement un gaz HFA, dans son réservoir 49. Ledit réservoir 49 est relié à un orifice de sortie 45 via un canal de flux secondaire 41. L'orifice de sortie 45 dudit système de génération de flux secondaire 40 est disposé axialement en aval dudit orifice de pulvérisation 35 dudit dispositif de pulvérisation nasale 100, et forme avec lui un angle alpha (a), par exemple de 45°. Ainsi, lors de l'actionnement manuel du dispositif de pulvérisation nasale 100, comme représenté sur la figure 13, le système de génération de flux secondaire 40, solidaire dudit dispositif de pulvérisation nasale 100, est également déclenché automatiquement pour produire une dose de gaz HFA qui est délivrée à travers l'orifice de sortie 45 simultanément à la distribution à travers l'orifice de pulvérisation 35 de la dose de médicament sous forme de spray. Le gaz HFA est dirigé par l'orifice de sortie 45 du système de génération de flux secondaire 40 selon ledit angle α par rapport à l'axe de l'orifice de pulvérisation 35. Ainsi, en aval dudit orifice de pulvérisation 35, les particules de liquides sont soumises non seulement à leurs vitesses initiales selon l'axe A mais également au flux de gaz secondaire provenant de l'orifice de sortie 45 du système de génération de flux secondaire 40. Les particules sont ainsi dirigées vers le plafond des cavités nasales.

Des tests comparatifs ont permis de démontrer que l'orientation dans la narine de l'utilisateur du dispositif de l'invention, en particulier lorsqu'il est réalisé selon les figures 12 et 13, peut avoir une influence sur l'efficacité du dispositif. Ces tests ont également mis en évidence des différences entre les hommes et les femmes, ainsi que des différences selon la profondeur d'insertion du dispositif dans la narine lors de l'utilisation.

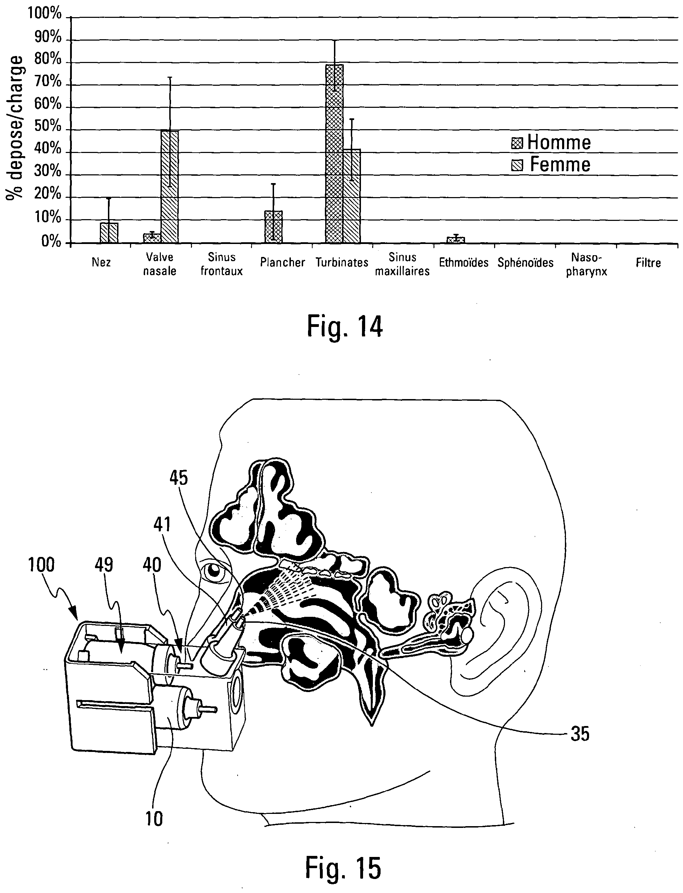

La figure 14 est ainsi un graphique représentant les dépôts de spray dans les différentes zones de la narine, pour un homme d'une part et pour une femme d'autre part, avec un dispositif réalisé et orienté comme sur les figures 12 et 13, c'est-à-dire avec le flux d'air déviant le spray vers le haut. Ici, l'angle alpha (a) qui s'est avéré optimal était de 30°. Le graphique montre que le dépôt de spray sur les éthmoïdes n'est que de 2,7% (+/- 0,1%) chez les hommes et quasi nul (0,2% +/- 0,1%) chez les femmes. Ceci peut notamment s'expliquer par le fait que dans certaines anatomies, les turbinates protègent les ethmoïdes, comme un écran, pour un spray dévié vers le haut.

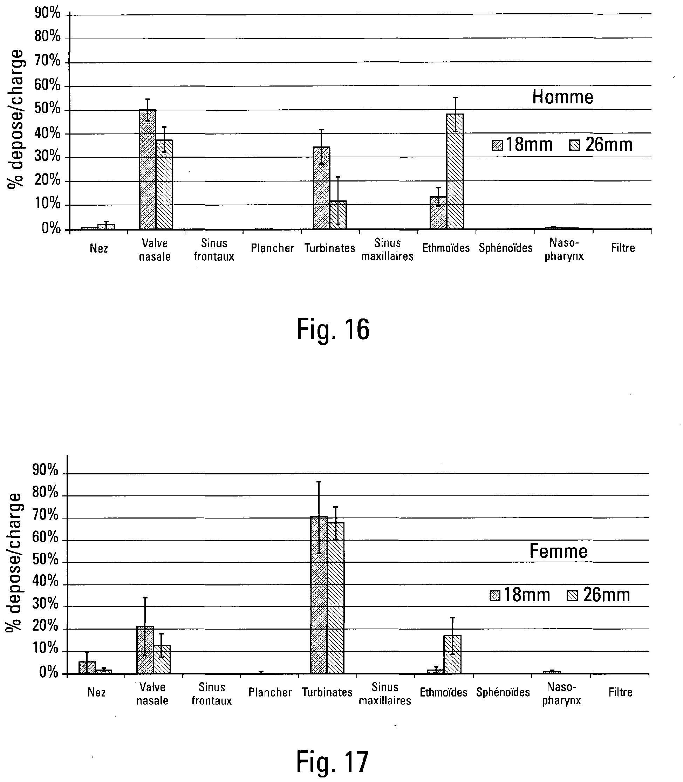

Des tests comparatifs ont donc été effectués avec une orientation différente, à savoir avec le flux d'air déviant le spray de manière latérale, comme illustré sur la figure 15. Ici, l'angle alpha (a) qui s'est avéré optimal était de 45°. Les figures 16 et 17 illustrent les résultats obtenus avec cette orientation de la figure 15, respectivement pour des modèles d'hommes et de femmes, et en comparant deux profondeurs d'insertion dans la narine, l'une de 18mm l'autre de 26mm.

On constate que pour le modèle d'homme, figure 16, le dépôt de spray sur les ethmoïdes est passé à 13,9% (+/- 3,7%) avec une profondeur d'insertion standard et même à 48,3% (+/- 7,1%) avec une profondeur d'insertion augmentée. Il en est de même pour le modèle de femme, figure 17, où le dépôt de spray sur les ethmoïdes est passé à 2,2% (+/- 1,3%) avec une profondeur d'insertion standard et à 17,4% (+/- 8,3%) avec une profondeur d'insertion augmentée.

Ces tests ont donc démontré qu'il est préférable de dévier le spray de manière latérale dans la narine, plutôt que vers le haut, et qu'une plus grande profondeur d'insertion du dispositif dans la narine est également bénéfique pour atteindre les ethmoïdes. Ainsi, une insertion d'au moins 15mm semble souhaitable, et une insertion de plus de 30mm pourrait présenter des risques d'inconfort et/ou de blessure pour l'utilisateur. Une plage de profondeur d'insertion avantageuse est donc comprise entre 15 et 30 mm.

Dans tous les cas précités, l'utilisation d'une source de gaz externe était nécessaire pour générer le ou les flux secondaire(s). Le système de génération de flux secondaire est alors dit actif. Cette source de gaz peut être une chasse d'air qui comprime de l'air à l'aide d'un piston, une petite bonbonne de gaz similaire à celles utilisées pour les inhalateurs à valve doseuse (MDI: Metered Dose Inhaler) contenant un gaz HFA, ou encore un compresseur d'air dans le cas d'un nébuliseur.

Une autre application de la présente invention concerne l'utilisation d'un dispositif de pulvérisation nasale associé à un système de génération de flux secondaire du type passif, c'est-à-dire dans lequel le flux secondaire n'est pas généré par une source externe, mais uniquement par l'inhalation de l'utilisateur. Dans ce cas, c'est donc l'utilisateur qui inhale qui va créer ce flux secondaire.

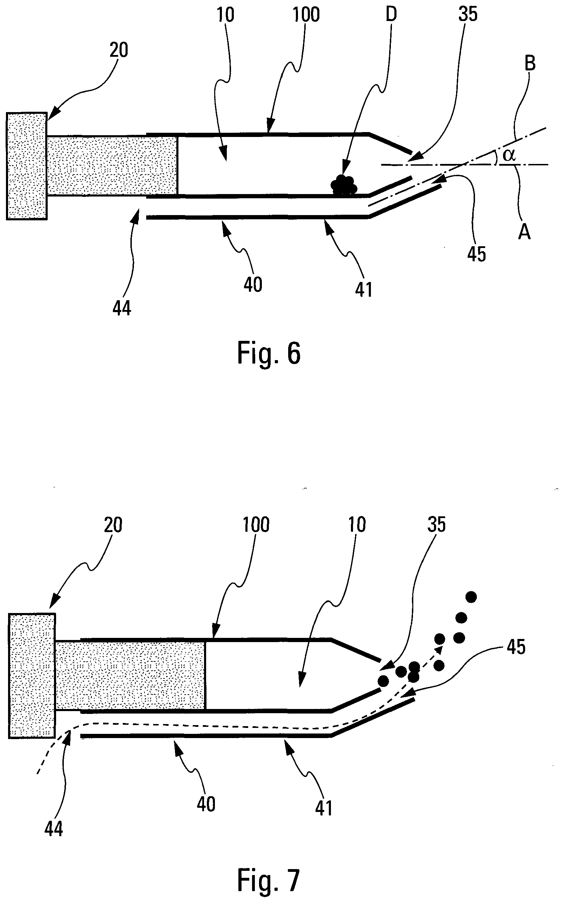

Les figures 6 et 7 illustrent de manière très schématique un mode de réalisation de l'invention avec un tel système de génération de flux secondaire du type passif. Le dispositif de pulvérisation nasale 100 comprend un réservoir 10 contenant une dose D de produit fluide, notamment sous forme de poudre. Un système de distribution 20 formant générateur d'aérosol comporte un piston coulissant dans ledit réservoir 10 entre une position avant actionnement, représentée sur la figure 6 et une position d'actionnement représentée sur la figure 7. Le dispositif de pulvérisation nasale 100 comporte à son extrémité axiale un orifice de pulvérisation 35, qui a avantageusement un diamètre de 2 mm, destiné à être introduit dans la narine du patient. Le dispositif de pulvérisation nasale 100 comporte en outre un système de génération de flux secondaire 40 comportant un canal de flux secondaire 41 pourvu d'un orifice d'entrée 44 et d'un orifice de sortie 45. L'orifice d'entrée 44 est relié à l'atmosphère et est destiné à faire pénétrer de l'air depuis l'extérieur dans le canal 41. L'orifice de sortie 45, qui a également avantageusement un diamètre de 2 mm, est destinée à administrer un flux d'air secondaire dans le spray issu de l'orifice de pulvérisation 35, afin de faire dévier les particules de spray de leur trajectoire sensiblement axiale. L'angle α est ici avantageusement de 35°.

Ainsi, lorsque l'utilisateur inhale par une inspiratoire nasale, le flux d'air inhalé pénètre dans le canal 41 puis est dirigé selon un angle de 35° à travers l'orifice de sortie 45. Durant cette phase d'inspiration, l'utilisateur appuie sur le piston du système de distribution 20, ce qui va générer un flux d'air comprimé dans le réservoir 10 pour aérosoliser et expulser la dose de poudre D sous forme de spray de particules à travers l'orifice de pulvérisation 35. Ainsi, en aval de l'orifice de pulvérisation 35, les particules de poudres sont soumises non seulement à leurs vitesses initiales selon l'axe A mais également au flux d'air secondaire provenant de l'orifice de sortie 45 du canal 41, qui les fait dévier de leur trajectoire selon une direction non axiale, comme visible sur la figure 7. Les particules de poudre sont ainsi dirigées vers le plafond des cavités nasales.

Un avantage de ce mode de réalisation est de ne pas utiliser de source externe de gaz ou d'air sous pression, et d'ajouter uniquement un élément mécanique simple, à savoir le canal 41, aux dispositifs de pulvérisation nasale existants.

Des tests comparatifs d'un dispositif standard avec un dispositif réalisé selon le mode de réalisation des figures 6 et 7 ont montré l'efficacité de l'invention, le dépôt de poudre au niveau des éthmoïdes obtenu avec le dispositif classique étant de 0%, alors qu'avec un flux d'air secondaire généré par l'inspiration de l'utilisateur, ce dépôt a été compris entre 3,5% et 7,8%, selon le débit du flux d'inhalation, qui est typiquement de l'ordre de 30L/min.

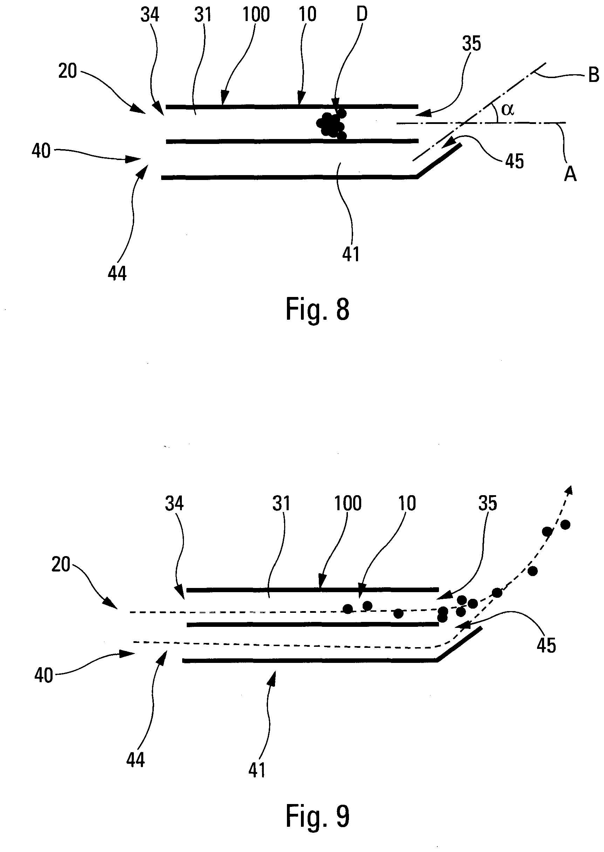

Les figures 8 et 9 illustrent un autre mode de réalisation de l'invention, avec un système de génération de flux secondaire du type passif.

Ici, le dispositif 100 comporte un réservoir 10 contenant une dose de poudre D, ce réservoir formant une conduite 31 comprenant un orifice d'entrée 34, destiné à faire pénétrer de l'air depuis l'extérieur vers l'intérieur de la conduite 31, et un orifice de pulvérisation 35, avantageusement d'un diamètre de 2 mm, destiné à expulser la dose de poudre sous forme de spray dans la narine de l'utilisateur. Cette conduite 31, avec ses orifices d'entrée 34 et de pulvérisation 35, forme donc le système de distribution 20 dans ce mode de réalisation. Le dispositif 100 comporte un canal 41 comprenant un orifice d'entrée 44, destiné à faire pénétrer de l'air depuis l'extérieur vers l'intérieur du canal 41, et un orifice de sortie 45, avantageusement d'un diamètre de 2 mm, destinée à délivrer un flux d'air secondaire dans le spray de particules afin de faire dévier ces particules de leur trajectoire sensiblement axiale. L'angle α est ici avantageusement de 35°.

Lors de la phase inspiratoire nasale de l'utilisateur, l'air inhalé traverse les deux orifices d'entrée 34 et 44 pour pénétrer respectivement dans la conduite 31 formant le réservoir 10 et dans le canal 41. L'air traversant la conduite 31 va transporter les particules de poudre jusqu'à l'orifice de pulvérisation 35. De manière simultanée, l'air traversant le canal 41 va être dirigée jusqu'à l'orifice de sortie 45. Ainsi, à la sortie de l'orifice de pulvérisation 35, les particules de poudres sont soumises non seulement à leur vitesse initiale sensiblement axiale, mais également au flux d'air secondaire provenant de l'orifice de sortie 45, qui les fait dévier de leur trajectoire initiale sensiblement axiale, comme visible sur la figure 9. Les particules de poudre sont ainsi dirigées vers le plafond des cavités nasales.

Un avantage de cette configuration est de ne pas utiliser de source externe de gaz ou d'air sous pression, et d'ajouter uniquement un élément mécanique simple, à savoir le canal 41, aux dispositifs de pulvérisation nasale existants. Par ailleurs, ce système est totalement passif, le système de distribution 20 et le système de génération de flux secondaire 40 étant chacun du type passif. De plus, la synchronisation du système de distribution 20 et du système de génération de flux secondaire 40 est réalisée automatiquement et naturellement par la seule inspiration de l'utilisateur.

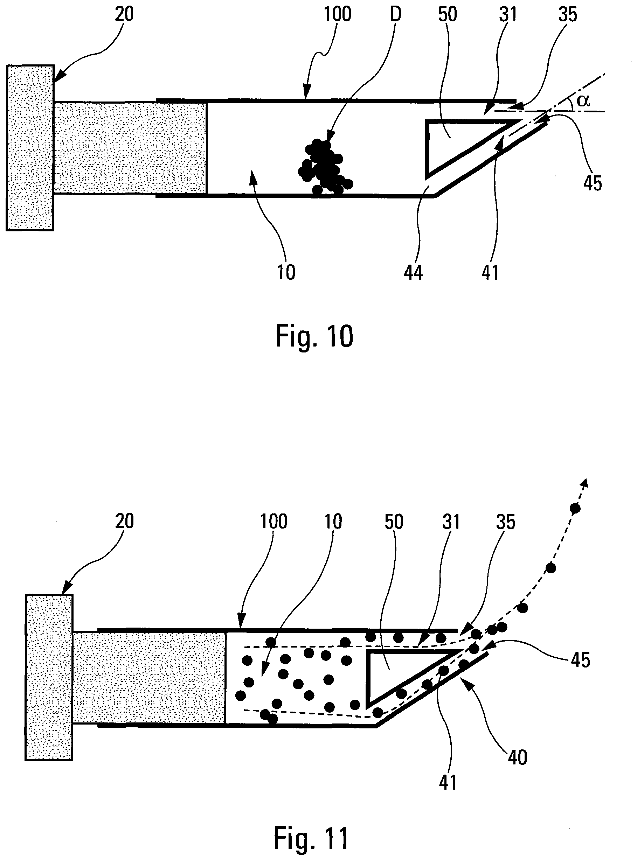

Les figures 10 et 11 illustrent encore un autre mode de réalisation de l'invention. Ce mode de réalisation diffère des modes de réalisation précédemment décrits, en ce que le flux secondaire n'est pas formé que de gaz, notamment d'air, mais comporte, comme le spray, un mélange de gaz, notamment d'air, et de produit fluide, notamment des particules de poudre.

Le dispositif de pulvérisation nasale 100 comprend un réservoir 10 contenant une dose D de produit fluide, notamment sous forme de poudre. Un système de distribution 20 formant générateur d'aérosol comporte un piston coulissant dans ledit réservoir 10 entre une position avant actionnement, représentée sur la figure 10 et une position d'actionnement représentée sur la figure 11. Contrairement au mode de réalisation des figures 6 et 7, le système de génération de flux secondaire 40 n'est ici pas séparé du système de distribution 20, mais au contraire, c'est le même piston qui va générer le spray qui sera expulsé à travers l'orifice de pulvérisation 35 et le flux d'air secondaire qui va être expulsé simultanément à travers l'orifice de sortie 45. Pour ce faire, le réservoir 10 contient un séparateur 50, disposé en amont de l'orifice de pulvérisation 35, qui, lors de l'actionnement, va dans le réservoir séparer le mélange poudre et air comprimé en deux flux, l'un cheminant dans une conduite axiale 31 vers l'orifice de pulvérisation 35, et l'autre cheminant dans un canal incliné 41 vers l'orifice de sortie 45. Avantageusement, l'orifice de pulvérisation 35 et l'orifice de sortie 45 ont chacun un diamètre d'environ 2 mm. L'inclinaison du canal 41, et donc l'angle α est avantageusement d'environ 35°

Ainsi, lors de l'actionnement, l'utilisateur pousse le piston dans le réservoir 10, ce qui comprime de l'air et permet d'aérosoliser la dose de poudre D dans le réservoir 10. Le séparateur 50 sépare cet aérosol en deux flux s'écoulant respectivement dans la conduite 31 et le canal 41. Ainsi, en aval de l'orifice de pulvérisation 35, les particules de poudre du spray sont soumises non seulement à leur vitesse initiale sensiblement axiale mais également au flux secondaire d'air et de particules provenant de l'orifice de sortie 45, qui les fait dévier de leur trajectoire axiale, comme visible sur la figure 11. Les particules de poudre sont ainsi dirigées vers le plafond des cavités nasales.

Un avantage de ce mode de réalisation est de ne pas utiliser de source externe de gaz ou d'air sous pression, et d'ajouter uniquement un élément mécanique simple, à savoir le séparateur 50, aux dispositifs de pulvérisation nasale existants.

Un avantage de la présente invention, quel que soit son mode de réalisation, est d'améliorer le dépôt de produit fluide dans les cavités nasales, notamment au niveau des éthmoïdes, sans avoir à modifier sensiblement les propriétés des sprays existants. En effet, la vitesse des particules, leurs tailles et d'autres propriétés du spray, peuvent ne pas être modifiées par la présente invention, l'ajout d'un flux secondaire après l'orifice de pulvérisation du spray classique étant suffisant pour atteindre les éthmoïdes. Ceci permet notamment de réaliser l'invention avec un dispositif petit et transportable dans la poche.

Il est à noter que les modes de réalisation des figures 6 et 11 décrits ci-dessus ne comportent pas de profil de pulvérisation en amont de l'orifice de pulvérisation, le produit fluide étant sous forme de poudre et l'aérosolisation se formant dans le réservoir 10. Il est toutefois entendu que chacun de ces modes de réalisation pourrait fonctionner aussi avec un tel profil de pulvérisation, associé par exemple à une pompe de distribution de produit fluide classique, notamment lorsque le produit fluide est sous forme liquide. Ceci peut par exemple être le cas avec les dispositifs à sprays, tel que décrit sur les figures 12 et 13, les dispositifs inhalateurs du type à bruine légère ou « soft mist », tel que le Respimat® de la société Boehringer-Ingelheim, et les nébuliseurs pneumatiques, ultrasoniques ou à tamis.

Par ailleurs, dans les modes de réalisation décrits ci-dessus, l'orifice de sortie 45 du système de génération de flux secondaire 40 est disposé au contact de l'orifice de pulvérisation 35, mais cet orifice de sortie pourrait être légèrement décalé, axialement et/ou latéralement, par rapport audit orifice de pulvérisation. En particulier, ces deux orifices 35 et 45 ne sont pas nécessairement formés sur une même pièce du dispositif de pulvérisation nasale 100. De plus, l'orientation du dispositif dans la narine peut être quelconque, avec toutefois une orientation préférée qui consiste à dévier le spray de manière latérale dans la narine.

La présente invention a été décrite en référence à plusieurs modes de réalisation avantageux, mais il est entendu qu'un homme du métier peut y apporter toutes modifications utiles, sans sortir du cadre de la présente invention telle que défini par les revendications annexées.

## Revendications

1. Dispositif de pulvérisation nasale de produit fluide (100) comportant un réservoir (10) contenant au moins une dose (D) de produit fluide, un système de distribution (20) pour pulvériser, à chaque actionnement du dispositif, une dose (D) de produit à travers un orifice de pulvérisation (35), générant un spray s'étendant axialement à partir dudit orifice de pulvérisation (35), **caractérisé en ce que** ledit dispositif (100) comprend un système de génération de flux secondaire (40) actionné simultanément audit système de distribution (20), ledit système de génération de flux secondaire (40) comportant un canal de flux secondaire (41) pourvu d'un orifice de sortie (45), ledit orifice de sortie (45) étant disposé en aval dudit orifice de pulvérisation (35) dans la direction axiale dudit spray, et ledit flux secondaire s'étendant à partir dudit orifice de sortie (45) selon un axe (B) formant un angle (a) par rapport à l'axe (A) dudit spray et coupant ledit axe (A), pour ainsi déformer et/ou dévier ledit spray.

2. Dispositif selon la revendication 1, dans lequel ledit flux secondaire dudit système de génération de flux secondaire (40) est formé par une source de gaz externe, notamment de gaz comprimé.

3. Dispositif selon la revendication 1, dans lequel ledit flux secondaire dudit système de génération de flux secondaire (40) est formé par le flux d'inhalation de l'utilisateur.

4. Dispositif selon l'une quelconque des revendications précédentes, dans lequel ledit flux secondaire dudit système de génération de flux secondaire (40) est constitué de gaz, notamment d'air.

5. Dispositif selon l'une quelconque des revendications 1 à 3, dans lequel ledit flux secondaire dudit système de génération de flux secondaire (40) comporte du gaz, notamment de l'air, mélangé avec du produit fluide, notamment des particules de poudre.

6. Dispositif selon l'une quelconque des revendications précédentes, dans lequel ledit angle (a) est compris entre 10° et 90°, avantageusement entre 20° et 80°, de préférence entre 30° et 70°, notamment environ 35°.

7. Dispositif selon l'une quelconque des revendications précédentes, dans lequel le diamètre dudit orifice de pulvérisation (35) est sensiblement identique au diamètre dudit orifice de sortie (45), notamment égal à environ 2 mm.

8. Dispositif selon l'une quelconque des revendications 1 à 6, dans lequel le diamètre dudit orifice de pulvérisation (35) est inférieur au diamètre dudit orifice de sortie (45).

9. Dispositif selon la revendication 8, dans lequel le diamètre dudit orifice de pulvérisation (35) est de 0,3 mm et le diamètre dudit orifice de sortie (45) est de 0,4 mm.

10. Dispositif selon l'une quelconque des revendications précédentes, dans lequel ledit canal de flux secondaire (41) dudit système de génération de flux secondaire (40) comporte un orifice d'entrée (44).

11. Dispositif selon la revendication 10, dans lequel ledit orifice d'entrée (44) est relié à une source de gaz, notamment d'air, comprimé.

12. Dispositif selon l'une quelconque des revendications 10, dans lequel ledit orifice d'entrée (44) est relié à l'atmosphère.

13. Dispositif selon l'une quelconque des revendications précédentes, dans lequel ledit produit fluide est un médicament sous forme de poudre.

14. Dispositif selon l'une quelconque des revendications 1 à 12, dans lequel ledit produit fluide est un médicament sous forme liquide.

15. Dispositif selon l'une quelconque des revendications précédentes, dans lequel ledit spray est composé de particules dont le diamètre est compris entre 1µm et 500µm.

16. Dispositif selon l'une quelconque des revendications précédentes, dans lequel ledit flux secondaire déforme et/ou dévie ledit spray de manière latérale dans la narine de l'utilisateur.

17. Dispositif selon l'une quelconque des revendications précédentes, dans lequel, lors de l'actionnement, ledit orifice de pulvérisation (35) est inséré dans la narine d'une profondeur comprise entre 15 et 30 mm.

18. Dispositif selon l'une quelconque des revendications précédentes, dans lequel, pour un modèle d'homme, au moins 10%, avantageusement environ 50%, du spray est déposé sur les ethmoïdes.

19. Dispositif selon l'une quelconque des revendications précédentes, dans lequel, pour un modèle de femme, au moins 2%, avantageusement environ 20%, du spray est déposé sur les ethmoïdes.

## Patentansprüche

1. Nasale Zerstäubungsvorrichtung für ein fluides Produkt (100), umfassend einen Behälter (10), der mindestens eine Dosis (D) fluides Produkt enthält, ein Ausgabesystem (20) zum Zerstäuben einer Produktdosis (D) durch eine Zerstäubungsöffnung (35) bei jeder Betätigung der Vorrichtung, wobei ein Spray erzeugt wird, das sich axial von der Zerstäubungsöffnung (35) verbreitet, **dadurch gekennzeichnet, dass** die Vorrichtung (100) ein System zur Erzeugung eines sekundären Stroms (40) umfasst, das gleichzeitig mit dem Ausgabesystem (20) betätigt wird, wobei das System zur Erzeugung eines sekundären Stroms (40) einen sekundären Strömungskanal (41) umfasst, der mit einer Ausgangsöffnung (45) versehen ist, wobei die Ausgangsöffnung (45) stromabwärts der Zerstäubungsöffnung (35) in Richtung axial des Sprays angeordnet ist und sich der sekundäre Strom von der Ausgangsöffnung (45) entlang einer Achse (B) verbreitet, die einen Winkel (a) in Bezug auf die Achse (A) des Sprays bildet und die Achse (A) schneidet, um so das Spray zu verformen und/oder abzulenken.

2. Vorrichtung nach Anspruch 1, wobei der sekundäre Strom des Systems zur Erzeugung eines sekundären Stroms (40) durch eine externe Gasquelle, insbesondere Druckgasquelle, gebildet ist.

3. Vorrichtung nach Anspruch 1, wobei der sekundäre Strom des Systems zur Erzeugung eines sekundären Stroms (40) durch den Inhalationsstrom des Benutzers gebildet wird.

4. Vorrichtung nach einem der vorhergehenden Ansprüche, wobei der sekundäre Strom des Systems zur Erzeugung eines sekundären Stroms (40) aus Gas, insbesondere Luft, besteht.

5. Vorrichtung nach einem der Ansprüche 1 bis 3, wobei der sekundäre Strom des Systems zur Erzeugung eines sekundären Stroms (40) Gas, insbesondere Luft, das/die mit fluidem Produkt, insbesondere Pulverteilchen, gemischt ist, umfasst.

6. Vorrichtung nach einem der vorhergehenden Ansprüche, wobei der Winkel (a) zwischen 10° und 90°, vorteilhafterweise zwischen 20° und 80°, vorzugsweise zwischen 30° und 70°, insbesondere bei etwa 35° liegt.

7. Vorrichtung nach einem der vorhergehenden Ansprüche, wobei der Durchmesser der Zerstäubungsöffnung (35) im Wesentlichen identisch zum Durchmesser der Ausgangsöffnung (45) ist und insbesondere etwa 2 mm entspricht.

8. Vorrichtung nach einem der Ansprüche 1 bis 6, wobei der Durchmesser der Zerstäubungsöffnung (35) kleiner als der Durchmesser der Ausgangsöffnung (45) ist.

9. Vorrichtung nach Anspruch 8, wobei der Durchmesser der Zerstäubungsöffnung (35) 0,3 mm beträgt und der Durchmesser der Ausgangsöffnung (45) 0,4 mm beträgt.

10. Vorrichtung nach einem der vorhergehenden Ansprüche, wobei der sekundäre Strömungskanal (41) des Systems zur Erzeugung eines sekundären Stroms (40) eine Eingangsöffnung (44) umfasst.

11. Vorrichtung nach Anspruch 10, wobei die Eingangsöffnung (44) mit einer Druckgasquelle, insbesondere Druckluftquelle, verbunden ist.

12. Vorrichtung nach einem der Ansprüche 10, wobei die Eingangsöffnung (44) mit Atmosphäre verbunden ist.

13. Vorrichtung nach einem der vorhergehenden Ansprüche, wobei das fluide Produkt ein Medikament in Form von Pulver ist.

14. Vorrichtung nach einem der Ansprüche 1 bis 12, wobei das fluide Produkt ein Medikament in flüssiger Form ist.

15. Vorrichtung nach einem der vorhergehenden Ansprüche, wobei das Spray aus Teilchen besteht, deren Durchmesser zwischen 1 µm und 500 µm liegt.

16. Vorrichtung nach einem der vorhergehenden Ansprüche, wobei der sekundäre Strom das Spray seitlich im Nasenloch des Benutzers verformt und/oder ablenkt.

17. Vorrichtung nach einem der vorhergehenden Ansprüche, wobei die Zerstäubungsöffnung (35) bei der Betätigung in eine Tiefe zwischen 15 und 30 mm in das Nasenloch eingeführt wird.

18. Vorrichtung nach einem der vorhergehenden Ansprüche, wobei sich bei einem durchschnittlichen Mann mindestens 10 %, vorteilhafterweise etwa 50 % des Sprays auf dem Siebbein ablagern.

19. Vorrichtung nach einem der vorhergehenden Ansprüche, wobei sich bei einer durchschnittlichen Frau mindestens 2 %, vorteilhafterweise etwa 20 % des Sprays auf dem Siebbein ablagern.

## Claims

1. A nasal fluid-spray device (100) comprising: a reservoir (10) containing at least one dose (D) of fluid; and a dispenser system (20) that, each time the device is actuated, sprays a dose (D) of fluid through a spray orifice (35), generating a spray that extends axially from said spray orifice (35); said device (100) being **characterized in that** it further comprises a generator system (40) for generating a secondary flow, which generator system is actuated simultaneously with said dispenser system (20), said secondary flow generator system (40) comprising a secondary flow channel (41) that is provided with an outlet orifice (45), said outlet orifice (45) being arranged downstream from said spray orifice (35) in the axial direction of said spray, and said secondary flow extending from said outlet orifice (45) along an axis (B) that forms an angle (α) relative to the axis (A) of said spray and that intersects said axis (A), thereby deforming and/or deflecting said spray.

2. A device according to claim 1, wherein said secondary flow of said secondary flow generator system (40) is formed by an external source of gas, in particular compressed gas.

3. A device according to claim 1, wherein said secondary flow of said secondary flow generator system (40) is formed by the user inhaling.

4. A device according to any preceding claim, wherein said secondary flow of said secondary flow generator system (40) is made up of gas, in particular of air.

5. A device according to any one of claims 1 to 3, wherein said secondary flow of said secondary flow generator system (40) comprises gas, in particular air, mixed with fluid, in particular particles of powder.

6. A device according to any preceding claim, wherein said angle (α) lies in the range 10° to 90°, advantageously in the range 20° to 80°, preferably in the range 30° to 70°, in particular about 35°.

7. A device according to any preceding claim, wherein the diameter of said spray orifice (35) is substantially identical to the diameter of said outlet orifice (45), in particular equal to about 2 mm.

8. A device according to any one of claims 1 to 6, wherein the diameter of said spray orifice (35) is less than the diameter of said outlet orifice (45).

9. A device according to claim 8, wherein the diameter of said spray orifice (35) is 0.3 mm and the diameter of said outlet orifice (45) is 0.4 mm.

10. A device according to any preceding claim, wherein said secondary flow channel (41) of said secondary flow generator system (40) includes an inlet orifice (44).

11. A device according to claim 10, wherein said inlet orifice (44) is connected to a source of gas, in particular of air, that is compressed.

12. A device according to any one of claims 10, wherein said inlet orifice (44) is connected to the atmosphere.

13. A device according to any preceding claim, wherein said fluid is a medication in powder form.

14. A device according to any one of claims 1 to 12, wherein said fluid is a medication in liquid form.

15. A device according to any preceding claim, wherein said spray is composed of particles having a diameter that lies in the range 1 µm to 500 µm.

16. A device according to any preceding claim, wherein said secondary flow deforms and/or deflects said spray sideways in the user's nostril.

17. A device according to any preceding claim, wherein, during actuation, said spray orifice (35) is inserted into the nostril to a depth that lies in the range 15 mm to 30 mm.

18. A device according to any preceding claim, wherein, for a man model, at least 10%, advantageously about 50%, of the spray is deposited on the ethmoids.

19. A device according to any preceding claim, wherein, for a woman model, at least 2%, advantageously about 20%, of the spray is deposited on the ethmoids.
